# EUROPÄISCHE PATENTSCHRIFT

(11) **EP 1 618 836 B1**
(45) Veröffentlichungstag und Bekanntmachung des Hinweises auf die Patenterteilung: **07.04.2010**
(21) Anmeldenummer: 05015894.8
(22) Anmeldetag: 21.07.2005
(51) Int. Cl.: A61B 1/267, A61B 5/00

(54) **Laryngoskop mit OCT**
Larygoscope with OCT
Laryngoscope avec tomographie à cohérence optique

(30) Priorität: 21.07.2004 DE 102004035269
(43) Veröffentlichungstag der Anmeldung: 25.01.2006
(73) Patentinhaber: Rowiak GmbH, 30419 Hannover (DE)
(72) Erfinder: Lüerßen, Kathrin, Dr. med., 30161 Hannover (DE); Lubatschowski, Holger, Dr., 30989 Gehrden (DE)
(74) Vertreter: Eisenführ, Speiser & Partner

(56) Entgegenhaltungen:
- WO-A-98/38907
- WO-A-99/57507
- FEIDCHTEIN F I ET AL: "Endoscopic applications of optical coherence tomography" OPTICS EXPRESS OPT. SOC. AMERICA USA, Bd. 3, Nr. 6, 14. September 1998 (1998-09-14), Seiten 257-270, XP002350131 ISSN: 1094-4087
- SERGEEV A M ET AL: "In vivo endoscopic OCT imaging of precancer and cancer states of human mucosa" OPTICS EXPRESS OPT. SOC. AMERICA USA, Bd. 1, Nr. 13, 22. Dezember 1997 (1997-12-22), Seiten 432-440, XP002350132 ISSN: 1094-4087

## Beschreibung

Die Erfindung betrifft ein Laryngoskop, umfassend einen lichtleitenden Abschnitt zum Einführen in die Mundhöhle eines Patienten und eine Beobachtungsvorrichtung zur Diagnostik mit sichtbarem Licht, einen Beleuchtungsstrahlengang zum Beleuchten eines Untersuchungsbereichs mit sichtbarem Beobachtungslichtes, einen Abbildungsstrahlengang zum Leiten des aus dem Untersuchungsbereich reflektierten Beobachtungslicht, eine erste optische Öffnung für den Eintritt des vom Untersuchungsbereich reflektierten Beobachtungslicht in das Laryngoskop, und eine zweite optische Öffnung zum Leiten des reflektierten Beobachtungslichtes zu einem Untersuchenden.

Laryngoskope der vorgenannten Art werden zur optischen Diagnostik in der Hals-Nasen-Ohren-Heilkunde verwendet und dienen insbesondere dazu, eine bildgebende Diagnostik der Stimmlippen vorzunehmen. Zu diesem Zweck wird das Laryngoskop in die Mundhöhle des Patienten eingeführt und soweit in Richtung des Rachenraums vorgeschoben, dass bei Absinken des Zungenrückens durch Ziehen an der Zunge aus dem Mund heraus nach unten eine Beobachtung der Stimmlippen möglich wird. Hierzu weist ein Laryngoskop üblicherweise an dem in die Mundhöhle eingeführten Ende eine Ablenkungseinrichtung zur Abwinklung der optischen Beobachtungsachse um etwa 70-90° Grad auf.

Bekannte Laryngoskope, wie beispielsweise das aus EP 0 901 772 A1 bekannte Laryngoskop, erlauben so eine Echtzeitbeobachtung der Stimmlippen. Viele pathologische Veränderungen der Stimmlippen lassen sich jedoch durch die damit mögliche statische Beobachtung der Stimmlippen nicht oder nicht sicher diagnostizieren.

Es ist HNO-ärztliche Routine, die Stimmlippen während Phonation (Tongebung) des Patienten zu stroboskopieren. Bei entsprechender Abstimmung der Stroboskop-Frequenz mit der durch die Tonhöhe bedingten Schwingfrequenz der Stimmlippen kann auf diese Weise eine Beobachtung des dynamischen Verhaltens der Stimmlippen ermöglicht werden. Eine Phasenverschiebung zwischen Stroboskopfrequenz und Schwingfrequenz der Stimmlippen ermöglicht eine verlangsamte Betrachtung des Schwingverhaltens der Stimmlippen.

Auch mit diesem Diagnoseverfahren können jedoch insbesondere in der Tiefe der Gewebestruktur der Stimmlippen liegende pathologische Veränderungen häufig nicht oder nicht sicher diagnostiziert werden.

Aus US 2002/0127632 A1 ist es bekannt, für die verbesserte Diagnostik im Bereich des Larynx eine Bilderfassung mittels optischer Kohärenztomographie durchzuführen. Zu diesem Zweck wird ein Katheter in den Zutrittskanal des Endoskops eingeführt. Ergebnisse, welche die Effizienz der Diagnostik mit einem solcherart modifizierten Endoskop belegen, sind aus den Artikeln Feidchtein F I ET AL: "Endoscopic applications of optical coherence tomography", Optics Express Opt. Soc. America USA, Bd. 3, Nr. 6, 14. September 1998 (1998-09-14), Seiten 257-270, XP002350131, ISSN: 1094-4087, Absatz 3 "EOCT of the larynx" und Sergeev A M ET AL: "In vivo endoscopic OCT imaging of precancer and cancer states of human mucosa", Optics Express Opt. Soc. America USA, Bd. 1, Nr. 13, 22. Dezember 1997 (1997-12-22), Seiten 432-440, XP002350132 ISSN: 1094-4087, Absatz 1 "Introduction", Absatz 2 "Implementation of endoscopic OCT device" bekannt.

Aus WO 99/57507 A ist eine weitere Vorrichtung bekannt, welche die Diagnostik mittels optischer Kohärenztomographie und gleichzeitiger visueller Beoachtung über parallel nebeneinandergeführte Beleuchtungs- und Beobachtungsstrahlengänge beschreibt.

Der Erfindung lag die Aufgabe zu Grunde, ein Laryngoskop zur verbesserten Diagnose pathologischer Veränderungen, insbesondere pathologischer Veränderungen der Stimmlippen, bereitzustellen.

Diese Aufgabe wird mit einem Laryngoskop gemäß Anspruch 1 gelöst.

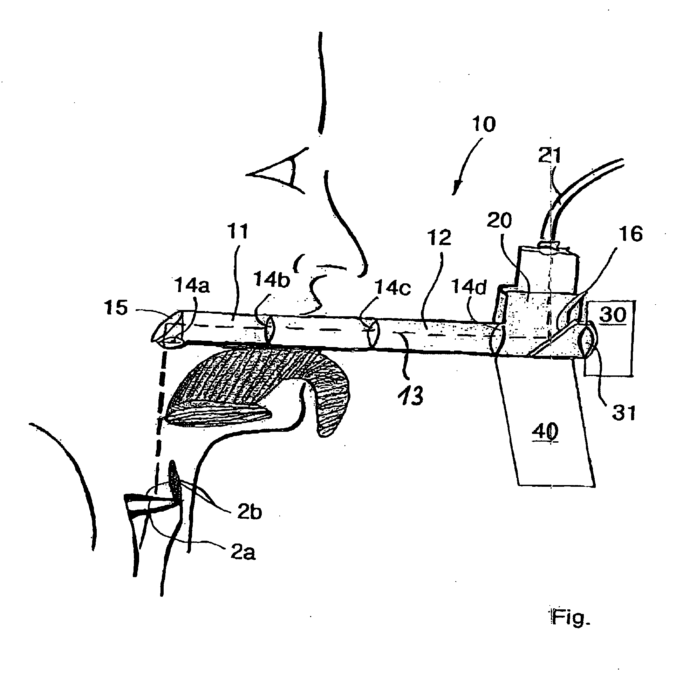

Das so fortgebildete Laryngoskop erlaubt die Abbildung des mit der Beobachtungsvorrichtung beobachteten Gewebes mittels der optischen Kohärenztomographie (OCT). Hierbei wird ein Messstrahl mit optisch kohärentem Licht auf einen Messpunkt geleitet und von den Gewebeschichten aus verschiedenen Tiefen zurückreflektiert. Auf Grund der unterschiedlichen zurückgelegten Pfadunterschiede in die einzelnen, höher oder tiefer gelegenen Gewebeschichten kann das zurückgestreute Licht durch Interferenzmessungen den einzelnen Gewebeschichten zugeordnet werden. Die Eindringtiefe in das Gewebe hängt dabei von der Wellenlänge der benutzen Strahlung ab, typischerweise benutzt man infrarote Strahlung oder Nahe zum Infrarotbereich liegende Strahlung, welche eine Eindringtiefe von etwa 3mm in das Gewebe aufweist.

Um mittels der optischen Kohärenztomographie auch größere Gewebeflächen darstellen zu können, kann der OCT-Messstrahl über eine solche größere Fläche geführt werden, und durch diese Abtastung ein Bild nach dem Scan-Prinzip erstellt werden.

Das solcher Art fortgebildete Laryngoskop erlaubt eine für die Diagnose äußerst hilfreiche Darstellung tieferer Gewebeschichten der Stimmbänder und kann daher auch pathologische Veränderungen in diesen tieferen Gewebeschichten darstellen.

Dabei können die einzelnen Strahlengänge des erfindungsgemäßen Laryngoskops insbesondere als Lichtwellenleiter ausgeführt sein.

Die am Laryngoskop angeordnete OCT-Vorrichtung erlaubt den Anschluss eines unmittelbar oder beabstandet zum Laryngoskop angeordneten OCT-Detektors und einer OCT-Strahlungsquelle mit gegebenenfalls daran ausgebildetem OCT-Scanner. Hierzu können der Beleuchtungsstrahlengang und/oder der Abbildungsstrahlengang der OCT-Vorrichtung über Leitungsmittel vom Laryngoskop zu dem OCT-Detektor und OCT-Scanner geleitet werden oder OCT-Scanner und OCT-Detektor können unmittelbar am Laryngoskop befestigt sein.

Eine erste vorteilhafte Ausführungsform besteht darin, dass der Beleuchtungsstrahlengang und der Abbildungsstrahlengang zumindest im Bereich des in die Mundhöhle einführbaren Abschnitts des Laryngoskops koaxial verlaufen. So kann die Beleuchtungsstrahlenquelle über einen halbdurchlässigen Spiegel in den Abbildungsstrahlengang eingekoppelt werden und folglich die Beobachtungsvorrichtung mit einem einzigen Strahlengang, der in einem einzigen Lichtwellenleiter untergebracht werden kann, ausgeführt werden.

Weiterhin ist es vorteilhaft, wenn der Kohärenzbeleuchtungsstrahlengang und der Kohärenzabbildungsstrahlengang zumindest im Bereich des in die Mundhöhle einführbaren Abschnitts des Laryngoskops koaxial verlaufen. Auf diese Art und Weise kann auch die OCT-Vorrichtung des Laryngoskops mit einem einzigen Strahlengang, der in einem einzigen Lichtwellenleiter untergebracht werden kann, ausgebildet werden.

Weiterhin ist es vorteilhaft, wenn mindestens ein Strahlengang der Beobachtungsvorrichtung und mindestens ein Strahlengang der OCT-Vorrichtung zumindest im Bereich des in die Mundhöhle einführbaren Abschnitts des Laryngoskops koaxial verlaufen. Diese Ausführungsform erlaubt eine insgesamt kompaktere Ausführung des erfindungsgemäßen Laryngoskops. Insbesondere ist es vorteilhaft, wenn alle vier Strahlengänge des Laryngoskops koaxial verlaufen, um auf diese Art und Weise alle erforderlichen Strahlengänge entlang einer einzigen optischen Achse, insbesondere in einem einzigen Lichtwellenleiter zusammenfassen zu können. Dies erlaubt eine besonders kompakte Ausbildung des in die Mundhöhle einzuführenden Abschnitt des Laryngoskops und folglich eine nur geringe Beeinträchtigung des Patienten durch die Untersuchung.

Das Laryngoskop kann weiter fortgebildet werden durch Mittel zur Zusammenführung und Trennung mindestens eines Strahlengangs der Beobachtungsvorrichtung und mindestens eines Strahlengangs der OCT-Vorrichtung. Durch diese Fortbildung ist es möglich, beispielsweise im außerhalb der Mundhöhle liegenden Abschnitt des Laryngoskops die Strahlengänge zusammenzuführen bzw. zu trennen und folglich einerseits einen schlanken Aufbau des in die Mundhöhle einzuführenden Abschnitts zu erzielen und anderseits einen konstruktiv einfach zu realisierenden Aufbau der Beobachtungsvorrichtung und des außerhalb der Mundhöhle liegenden OCT-Vorrichtungsteils zu erzielen.

Dabei ist es besonders vorteilhaft, wenn die Mittel zur Zusammenführung und Trennung der Strahlengänge einen pleochroitischen, insbesondere einen dichroitischen, Strahlteiler umfassen. Unter einem solchen Strahlteiler versteht man ein optisches Element, welches in Abhängigkeit der Wellenlänge der durch das Element hindurchlaufenden Strahlung unterschiedliche optische Eigenschaften aufweist. Insbesondere kann ein solches optisches Element ausgebildet sein, um Strahlung einer bestimmten Wellenlänge unter einem bestimmten Winkel zu reflektieren und Strahlung einer anderen Wellenlänge nicht zu reflektieren. In dem Falle des erfindungsgemäßen Laryngoskops ist insbesondere ein dichroitischer Strahlteiler, der also auf zwei unterschiedliche Wellenlängen oder Wellenlängenbereiche verschieden reagiert, vorteilhaft, um solcher Art die typischerweise im nichtsichtbaren Infrarotbereich liegende OCT-Strahlung von der Lichtstrahlung der Beobachtungsvorrichtung im sichtbaren Bereich zu trennen.

Weiterhin ist es vorteilhaft, wenn die OCT-Vorrichtung ein an dem Laryngoskop befestigbares OCT-Modul mit einem OCT-Scanner und einem OCT-Detektor umfasst. Ein solches Laryngoskop ist besonders einfach zu handeln und erlaubt einem Untersuchenden eine einfache Zielausrichtung, Fokussierung und Handhabung des Laryngoskops.

Weiterhin ist es vorteilhaft, wenn der Arbeitsabstand der OCT-Vorrichtung etwa mit dem Arbeitsabstand der Beobachtungsvorrichtung übereinstimmt und insbesondere der Distanz zwischen der ersten optischen Öffnung des in den Mundraum eines Patienten eingeführten Laryngoskops und den Stimmlippen des Patienten entspricht. Bei Untersuchungen im Rachenbereich ist es regelmäßig problematisch, diagnostische Instrumente unmittelbar an den Untersuchungsbereich heranzuführen, da hierdurch häufig beim untersuchten Patienten ein Schluckreflex oder insbesondere der Würgereflex ausgelöst wird. Es ist daher insbesondere vorteilhaft, wenn eine Beobachtung des Untersuchungsbereichs aus größerer Distanz möglich ist. So ist es beispielsweise insbesondere vorteilhaft, wenn ein Laryngoskop mit seinem in die Mundhöhle einzuführenden Ende nur bis an den Anfang des Rachenraums vorgeschoben werden muss, um dann eine Beobachtung der Stimmlippen über eine Distanz von ca. 4 bis 8 cm zu ermöglichen. Der Arbeitsabstand des Laryngoskops ist daher vorteilhafterweise für diese Distanz ausgebildet.

Bei einer weiteren Fortbildung des erfindungsgemäßen Laryngoskops stimmt der Tiefenschärfebereich der Beobachtungsvorrichtung im Wesentlichen mit dem Arbeitsbereich der OCT-Vorrichtung überein.

Unter Arbeitsabstand ist in diesem Zusammenhang der Abstand zwischen der ersten optischen Öffnung und dem Untersuchungsbereich zu verstehen. Unter Arbeitsbereich ist in diesem Zusammenhang der Toleranzbereich zu verstehen, innerhalb dessen der Arbeitsabstand variiert werden kann, ohne dass die Qualität der Abbildung sich maßgeblich verringert. Die Grenzen des einzustellenden Distanzbereichs ergeben sich also rechnerisch aus dem Arbeitsabstand plus/minus der Hälfte des Arbeitsbereichs.

Die derzeit bekannten optischen Kohärenztomographen können typischerweise nur in einem Arbeitsbereich von 2 - 5 mm arbeiten. Es ist daher erforderlich, den Arbeitsabstand, also regelmäßig den Abstand zwischen der ersten optischen Öffnung und dem Untersuchungsbereich, mit einer Genauigkeit von ca. 2 - 5mm einzustellen, um eine genau Abbildung mittels der OCT zu erzielen.

Die genaue Einstellung des Arbeitsabstandes kann insbesondere dadurch erfolgen, dass ein Untersuchender mittels Verstellung optischer Fokussierungsmittel das mit der Beobachtungsvorrichtung gewonnene Bild scharfstellt, wobei diese Fokussierungsmittel gleichermaßen auf die OCT-Strahlung wirken und folglich den Arbeitsabstand der OCT-Vorrichtung ändern. Ein Alternative oder Ergänzung hierzu ist, bei unveränderter Fokussierung und folglich unverändertem Arbeitsabstand durch Bewegen des Laryngoskops die Distanz zwischen Untersuchungsbereich und Laryngoskop so einzustellen, dass das mit der Beobachtungsvorrichtung gewonnene Bild scharf abgebildet wird und folglich der korrekte Arbeitsabstand erzielt wird.

Wird hierbei der Tiefenschärfebereich der Beobachtungsvorrichtung etwa gleich dem Arbeitsbereich der OCT-Vorrichtung gewählt, so ist bei scharfer Abbildung des Untersuchungsbereichs mit der Beobachtungsvorrichtung stets gleichzeitig sichergestellt, dass der korrekte Arbeitsabstand für die OCT-Vorrichtung eingestellt ist.

Diese Ausführungsform ist insbesondere dann vorteilhaft, wenn die Strahlengänge der Beobachtungsvorrichtung und der OCT-Vorrichtung koaxial verlaufen und folglich aus einer gemeinsamen optischen Öffnung auf den Untersuchungsbereich treffen bzw. durch diese Öffnung von dem Untersuchungsbereich in das Laryngoskop eingeleitet werden. In diesem Fall kann die Distanz zwischen der ersten optischen Öffnung und dem Untersuchungsbereich, die den Arbeitsabstand darstellt, durch Fokussierung und/oder Bewegung des Laryngoskops eingestellt werden und stellt dann sicher, dass die Distanz nicht außerhalb des Arbeitsbereichs der OCT-Vorrichtung liegt.

Eine weitere Fortbildung des erfindungsgemäßen Laryngoskops ist gekennzeichnet durch Mittel zum Beeinflussen des Tiefenschärfebereichs der Beobachtungsvorrichtung, insbesondere zum Angleichen des Tiefenschärfebereichs an den Arbeitsbereich der OCT-Vorrichtung. Mit solchen Mitteln kann der Tiefenschärfebereich einer Optik eines Laryngoskops insbesondere verringert werden, um solcher Art an den nur geringen Arbeitsbereich der OCT-Vorrichtung angepasst zu werden.

Dabei ist es insbesondere vorteilhaft, wenn die Wirkung der Mittel zum Beeinflussen des Tiefenschärfebereichs ausgeschaltet werden kann. Das Ausschalten der Wirkung kann beispielsweise durch Herausbewegen oder Hereinbewegen optischer Elemente aus dem bzw. in den Strahlengang erfolgen. Durch diese Fortbildung kann eine zunächst konventionelle Untersuchung mittels des Laryngoskops bei ausgeschalteter Wirkung erfolgen und hierauf folgend kann die Wirkung eingeschaltet werden und die erforderliche Justierung bzw. Fokussierung zur Diagnostik mittels der OCT-Vorrichtung kann erfolgen.

Bei den beiden vorgenannten Ausführungsformen ist es insbesondere vorteilhaft, wenn die Mittel zum Beeinflussen des Tiefenschärfebereichs eine Apertur im Abbildungsstrahlengang umfassen, die so groß gewählt ist, dass der Tiefenschärfebereich der Beobachtungsvorrichtung im Wesentlichen mit dem Arbeitsbereich der OCT-Vorrichtung übereinstimmt, insbesondere nicht größer ist als der Arbeitsbereich der OCT-Vorrichtung. Eine solche Apertur kann beispielsweise eine Blende umfassen, deren Durchtrittsöffnungsdurchmesser entsprechend des gewünschten Tiefenschärfebereichs gewählt ist. Eine solche Blende kann dann gegebenenfalls aus dem Strahlengang herausgeschwenkt werden oder durch eine kleinere Blende ersetzt werden, um die Beeinflussung des Tiefenschärfebereichs aufzuheben.

Eine weitere Fortbildung der vorgenannten Ausführungsformen mit Mitteln zum Beeinflussen des Tiefenschärfebereichs umfasst Mittel zur Erhöhung der Gesamtvergrößerung der Beobachtungsvorrichtung, die so gewählt sind, dass der Tiefenschärfebereich der Beobachtungsvorrichtung im Wesentlichen mit dem Arbeitsbereich der OCT-Vorrichtung übereinstimmt, insbesondere nicht größer ist als der Arbeitsbereich der OCT-Vorrichtung. Bei dieser Fortbildung ist einerseits eine sichere Einstellung auf den Arbeitsbereich der OCT-Vorrichtung möglich, wie zuvor beschrieben, und andererseits wird die Einstellung dieses Arbeitsbereichs sehr präzise möglich, indem eine vergrößerte Abbildung des Untersuchungsbereichs ermöglicht wird. Die solcher Art ausgebildeten Mittel zum Beeinflussen des Tiefenschärfebereichs können beispielsweise in Gestalt einer oder zweier optischer Linsen ausgebildet werden, die auch gegebenenfalls zur Ausschaltung dieser Wirkung aus dem Strahlengang herausbewegt werden können.

Dabei ist es insbesondere vorteilhaft, wenn die Mittel zur Erhöhung der Gesamtvergrößerung ausgebildet sind für eine stufenlose Erhöhung der Gesamtvergrößerung. Eine solche Zoomoptik erlaubt eine an die jeweilige unterschiedliche Untersuchungsgegebenheit und Anatomie angepasste Einstellung der Gesamtvergrößerung.

Eine weitere vorteilhafte Fortbildung des erfindungsgemäßen Laryngoskops umfasst Mittel zur Vergrößerung des Tiefenschärfebereichs und/oder des Gesichtsfeldes der Beobachtungsvorrichtung. Mit dieser Fortbildung ist zur leichteren Orientierung und zur verbesserten konventionellen Diagnostik eine bessere Beobachtung des Untersuchungsbereichs mit erleichterter Fokussierung bzw. größerem Beobachtungsbereich möglich. Die solcher Art hinzugefügten Mittel zur Vergrößerung des Tiefenschärfebereichs können beispielsweise mittels einer kleineren Blende ausgeführt sein.

Dabei ist es insbesondere vorteilhaft, wenn die Wirkung der Mittel zur Vergrößerung des Tiefenschärfebereichs und/oder des Gesichtsfeldes der Beobachtungsvorrichtung ausgeschaltet werden kann. Auf diese Weise kann nach einer grundsätzlichen Orientierung und konventionellen Untersuchung mittels des Laryngoskops die Wirkung der Vergrößerungsmittel ausgeschaltet werden, beispielsweise indem die entsprechenden Mittel aus dem Strahlengang herausbewegt werden, und nachfolgend eine Untersuchung mittels der OCT-Vorrichtung erfolgen.

Eine weitere Fortbildung ist gekennzeichnet durch Mittel zur Erzeugung eines sichtbaren Pilotstrahls, der auf den Untersuchungsbereich der OCT-Vorrichtung gerichtet ist. Da die OCT-Strahlung typischerweise im nicht-sichtbaren Bereich arbeitet, ist es für einen Untersuchenden nicht erkennbar, an welcher Stelle des Untersuchungsbereichs die OCT-Diagnostik erfolgt. Es ist daher vorteilhaft, wenn ein Pilotstrahl koaxial zum OCT-Strahl angeordnet wird und auf diese Weise den OCT-Messpunkt markiert. Dabei kann der Pilotstrahl auch in solcher Weise ausgebildet sein, dass er den vom OCT-Messstrahl abgescannten Bereich markiert. Dies kann durch flächige Beleuchtung des OCT-Messbereichs erfolgen, oder beispielsweise durch Umrandung dieses OCT-Messbereichs.

Es ist insbesondere vorteilhaft, wenn die zweite optische Öffnung mit einem Bilderfassunggerät, insbesondere einer CCD-Kamera, zusammenwirkt. Auf diese Weise kann eine Speicherung der mit der Beobachtungsvorrichtung erfassten Bilder erfolgen und darüber hinaus eine Abbildung dieser Bilder an einem vom Laryngoskops beabstandeten Ort ermöglicht werden.

Zu diesem Zweck ist es weiterhin vorteilhaft, wenn ein Bildwiedergabegerät zur Darstellung des mit dem Bilderfassunggerät aufgenommenen Bildes vorhanden ist. Ein solches Bildwiedergabegerät kann beispielsweise in Gestalt eines Bildschirms oder eines Projektors bereitgestellt werden und erlaubt die Betrachtung der mit der Beobachtungsvorrichtung erfassten Bilder durch mehrer Beobachter.

Es insbesondere vorteilhaft, wenn die zweite optische Öffnung als Okular ausgebildet ist, um den Untersuchungsbereich unmittelbar mit dem Auge eines Untersuchenden betrachten zu können. Dies ermöglicht die Benutzung des erfindungsgemäßen Laryngoskops in konventioneller Art und Weise und erlaubt daher insbesondere solchen Untersuchenden, die über langjährige Praxis mit konventionellen Laryngoskopen verfügen, eine einfache ergänzende Anwendung der mit dem erfindungsgemäßen Laryngoskop erzielbaren zusätzlichen Diagnostik durch OCT-Abbildungen.

Schließlich ist es vorteilhaft, wenn der Beleuchtungsstrahlengang mit einem Stroboskop zur Beleuchtung des Untersuchungsbereichs verbindbar ist. Auf diese Art und Weise erlaubt das erfindungsgemäße Laryngoskops die bekannte laryngo-stroboskopische Diagnostik und ergänzend hierzu die konventionelle statische Beobachtung und die erfindungsgemäße OCT-Abbildung der Stimmlippen.

Ein bevorzugte Ausführungsform wird anhand der beigefügten Figur beschrieben. Die Figur zeigt eine teilgeschnittene Seitansicht des erfindungsgemäßen Laryngoskops in einer in die Mundhöhle eines Patienten eingeführten Lage.

Die Abbildung zeigt einen Patienten 1 mit Stimmlippen 2a, b und einem in die Mundhöhle teilweise eingeführten Laryngoskops 10.

Das Laryngoskop 10 weist einen in die Mundhöhle einführbaren ersten Abschnitt 11 auf, der im Wesentlichen rohrförmig ausgebildet ist. Der erste Abschnitt 11 verlängert sich nach außerhalb der Mundhöhle in einen zweiten rohrförmigen Abschnitt 12.

Innerhalb der Abschnitte 11, 12 verläuft eine optische Achse 13, entlang welcher der Beleuchtungsstrahlengang und der Abbildungsstrahlengang der Beobachtungsvorrichtung und der Kohärenzbeleuchtungsstrahlengang und der Kohärenzabbildungsstrahlengang der OCT-Vorrichtung koaxial verlaufen. Diese vier Strahlengänge werden durch mehrere entlang der optischen Achse 13 beabstandet voneinander angeordnet optische Elemente 14a - d geführt.

An dem in dem in die Mundhöhle eingeführten Ende des Abschnitts 11 ist ein Strahlablenkungsvorrichtung 15, beispielsweise ein Prisma, angeordnet, welches den Strahl um etwa 70 Grad - 90 Grad ablenkt.

Am außerhalb des Patienten liegenden Ende des Laryngoskops ist im Strahlengang entlang der optischen Achse 13 ein dichroitischer Strahlteiler 16 in Gestalt eines Spiegels angeordnet, der die OCT-Strahlung um 90 Grad ablenkt und die Strahlung im sichtbaren Lichtbereich unabgelenkt passieren lässt. Hierdurch werden der Kohärenzbeleuchtungsstrahlengang und der Kohärenzabbildungsstrahlengang um 90 Grad abgelenkt und in ein OCT-Modul 20 geleitet.

Das OCT-Modul 20 ist über eine Faseroptik 21 mit einer OCT-Strahlungsquelle und einen OCT-Detektor (nicht abgebildet) verbunden. In dem OCT-Modul 20 ist ein OCT-Scanner zur Ablenkung und Abtastung eines OCT-Untersuchungsbereichs und eine Teleskopoptik zur Anpassung der OCT-Strahlung an die Optik des Abbildungsstrahlengangs in Abschnitt 11,12 angeordnet, der die von den Stimmlippen 2a, b reflektierte OCT-Strahlung erfasst.

Das den dichroitischen Strahlteiler 16 durchdringende Licht im sichtbaren Bereich wird durch eine Okularlinse 31 einer CCD-Kamera 30 zugeführt und erlaubt die Abbildung der Stimmlippen 2a, b auf einem Bildschirm (nicht dargestellt). Die CCD-Kamera 30 ist lösbar am Laryngoskop befestigt, um auch eine unmittelbare Betrachtung der Stimmlippen 2a, b durch das Okular 31 für den Untersuchenden zu erlauben.

In Bezug auf die optische Achse 13 gegenüberliegend zum OCT-Modul 20 ist ein Handgriff 40 angeordnet, der eine einfache Handhabung und Ausrichtung des Laryngoskops durch den Untersuchenden erlaubt.

## Patentansprüche

1. Laryngoskop, umfassend
- einen lichtleitenden Abschnitt (11) zum Einführen in die Mundhöhle eines Patienten und
- eine Beobachtungsvorrichtung (14a-d, 31. 30) zur Diagnostik mit sichtbarem Licht, umfassend
- einen Beleuchtungsstrahlengang (13) zum Beleuchten eines Untersuchungsbereichs (2a,b) mit sichtbarem Beobachtungslichtes,
- einen Abbildungsstrahlengang (13) zum Leiten des aus dem Untersuchungsbereich reflektierten Beobachtungslicht,
- eine erste optische Öffnung (14a) für den Eintritt des vom Untersuchungsbereich reflektierten Beobachtungslicht in das Laryngoskop, und
- eine zweite optische Öffnung (31) zum Leiten des reflektierten Beobachtungslichtes zu einem Untersuchenden,
- eine OCT-Vorrichtung (14a-d, 16) zur Diagnostik mittels optischer Kohärenztomographie, umfassend
- einen Kohärenzbeleuchtungsstrahlengang (13) zum Beleuchten eines Untersuchungsbereichs mit der kohärenten Strahlung, und
- einen Kohärenzabbildungsstrahlengang (13) zum Leiten der aus dem Untersuchungsbereich reflektierten kohärenten Strahlung zu einem OCT-Modul (20) zum Erzeugen einer Gewebeschichtaufnahme aus dem Untersuchungsbereich,
**dadurch gekennzeichnet, dass** der Arbeitsabstand der OCT-Vorrichtung etwa mit dem Arbeitsabstand der Beobachtungsvorrichtung übereinstimmt und insbesondere der Distanz zwischen der ersten optischen Öffnung des in den Mundraum eines Patienten eingeführten Laryngoskops und den Stimmlippen des Patienten entspricht und der Tiefenschärfebereich der Beobachtungsvorrichtung im Wesentlichen mit dem Arbeitsbereich der OCT-Vorrichtung übereinstimmt.

2. Laryngoskop nach Anspruch 1,
**dadurch gekennzeichnet, dass** der Beleuchtungsstrahlengang (13) und der Abbildungsstrahlengang (13) zumindest im Bereich des in die Mundhöhle einführbaren Abschnitts (11) des Laryngoskops koaxial verlaufen.

3. Laryngoskop nach Anspruch 1 oder 2,
**dadurch gekennzeichnet, dass** der Kohärenzbeleuchtungsstrahlengang (13) und der Kohärenzabbildungsstrahlengang (13) zumindest im Bereich des in die Mundhöhle einführbaren Abschnitts (11) des Laryngoskops koaxial verlaufen.

4. Laryngoskop nach einem der vorhergehenden Ansprüche,
**dadurch gekennzeichnet, dass** mindestens ein Strahlengang (13) der Beobachtungsvorrichtung und mindestens ein Strahlengang (13) der OCT-Vorrichtung zumindest im Bereich des in die Mundhöhle einführbaren Abschnitts (11) des Laryngoskops koaxial verlaufen.

5. Laryngoskop nach einem der vorhergehenden Ansprüche,
**gekennzeichnet durch** Mittel (16) zur Zusammenführung und Trennung mindestens eines Strahlengangs (13) der Beobachtungsvorrichtung und mindestens eines Strahlengangs (13) der OCT-Vorrichtung.

6. Laryngoskop nach Anspruch 5,
**dadurch gekennzeichnet, dass** die Mittel zur Zusammenführung und Trennung der Strahlengänge einen pleochroitischen, insbesondere einen dichroitischen, Strahlteiler (16) umfassen.

7. Laryngoskop nach einem der vorhergehenden Ansprüche,
**dadurch gekennzeichnet, dass** die OCT-Vorrichtung weiterhin ein an dem Laryngoskop befestigbares OCT-Modul (30) mit einem OCT-Scanner und einer Teleskopoptik umfasst.

8. Laryngoskop nach einem der vorhergehenden Ansprüche,
**gekennzeichnet durch** Mittel (14a-d) zum Beeinflussen des Tiefenschärfebereichs der Beobachtungsvorrichtung, insbesondere zum Angleichen des Tiefenschärfebereichs an den Arbeitsbereich der OCT-Vorrichtung.

9. Laryngoskop nach Anspruch 8,
**dadurch gekennzeichnet, dass** die Wirkung der Mittel zum Beeinflussen des Tiefenschärfebereichs ausgeschaltet werden kann.

10. Laryngoskop nach einem der Ansprüche 8 bis 9,
**dadurch gekennzeichnet, dass** die Mittel zum Beeinflussen des Tiefenschärfebereichs eine Apertur im Abbildungsstrahlengang umfassen, die so groß gewählt ist, dass der Tiefenschärfebereich der Beobachtungsvorrichtung im Wesentlichen mit dem Arbeitsbereich der OCT-Vorrichtung übereinstimmt, insbesondere nicht größer ist als der Arbeitsbereich der OCT-Vorrichtung.

11. Laryngoskop nach einem der Ansprüche 8 bis 10,
**dadurch gekennzeichnet, dass** die Mittel zum Beeinflussen des Tiefenschärfebereichs Mittel (31) zur Erhöhung der Gesamtvergrößerung der Beobachtungsvorrichtung umfassen, die so gewählt sind, dass der Tiefenschärfebereich der Beobachtungsvorrichtung im Wesentlichen mit dem Arbeitsbereich der OCT-Vorrichtung übereinstimmt, insbesondere nicht größer ist als der Arbeitsbereich der OCT-Vorrichtung.

12. Laryngoskop nach Anspruch 11,
**dadurch gekennzeichnet, dass** Mittel zur Erhöhung der Gesamtvergrößerung ausgebildet sind für eine stufenlose Erhöhung der Gesamtvergrößerung.

13. Laryngoskop nach einem der vorhergehenden Ansprüche,
**gekennzeichnet durch** Mittel (31) zur Vergrößerung des Tiefenschärfebereichs und/oder des Gesichtsfeldes der Beobachtungsvorrichtung.

14. Laryngoskop nach Anspruch 13,
**dadurch gekennzeichnet, dass** die Wirkung der Mittel zur Vergrößerung des Tiefenschärfebereichs und/oder des Gesichtsfeldes der Beobachtungsvorrichtung ausgeschaltet werden kann.

15. Laryngoskop nach einem der vorhergehenden Ansprüche,
**gekennzeichnet durch** Mittel zur Erzeugung eines sichtbaren Pilotstrahls, der auf den Untersuchungsbereich der OCT-Vorrichtung gerichtet ist.

16. Laryngoskop nach einem der vorhergehenden Ansprüche,
**dadurch gekennzeichnet, dass** die zweite optische Öffnung (31) mit einem Bilderfassunggerät (30), insbesondere einer CCD-Kamera, zusammenwirkt.

17. Laryngoskop nach Anspruch 16,
**gekennzeichnet durch** ein Bildwiedergabegerät zur Darstellung des mit der Bilderfassunggerät aufgenommenen Bildes.

18. Laryngoskop nach einem der vorhergehenden Ansprüche,
**dadurch gekennzeichnet, dass** die zweite optische Öffnung (31) als Okular ausgebildet ist, um den Untersuchungsbereich unmittelbar mit dem Auge eines Untersuchenden betrachten zu können.

19. Laryngoskop nach einem der vorhergehenden Ansprüche,
**dadurch gekennzeichnet, dass** der Beleuchtungsstrahlengang (13) mit einem Stroboskop zur Beleuchtung des Untersuchungsbereichs verbindbar ist.

## Claims

1. Laryngoscope, comprising
- a light guiding portion (11) for insertion into a patient's oral cavity and
- an observation device (14a-d, 31, 30) for diagnostics with visible light, comprising
- an illumination beam path (13) for illuminating an examination area (2a, b) with visible observation light,
- an imaging beam path (13) for guiding the observation light reflected from the examination area,
- a first optical opening (14a) through which the observation light reflected from the examination area enters the laryngoscope, and
- a second optical opening (31) for guiding the reflected observation light to an examiner,
- an OCT device (14a-d, 16) for diagnostics by means of optical coherence tomography, comprising
- a coherence illumination beam path (13) for illuminating an examination area with the coherent radiation, and
- a coherence imaging beam path (13) for guiding the coherent radiation reflected from the examination area to an OCT module (20) for generating a tissue layer image of the examination area,
**characterised in that** the operating distance of the OCT device is approximately equal to the operating distance of the observation device and is equal, in particular, to the distance between the first optical opening of the laryngoscope inserted into a patient's oral cavity and the patient's vocal folds and the depth of focus range of the observation device is substantially equal to the operating range of the OCT device.

2. Laryngoscope according to claim 1, **characterised in that** the illumination beam path (13) and the imaging beam path (13) run coaxially at least in the region of the portion (11) of the laryngoscope that is insertable into the oral cavity.

3. Laryngoscope according to claim 1 or 2, **characterised in that** the coherence illumination beam path (13) and the coherence imaging beam path (13) run coaxially at least in the region of the portion (11) of the laryngoscope that is insertable into the oral cavity.

4. Laryngoscope according to any one of the preceding claims,
**characterised in that** at least one beam path (13) of the observation device and at least one beam path (13) of the OCT device run coaxially at least in the region of the portion (11) of the laryngoscope that is insertable into the oral cavity.

5. Laryngoscope according to any one of the preceding claims,
**characterised by** means (16) for combining and splitting at least one beam path (13) of the observation device and at least one beam path (13) of the OCT device.

6. Laryngoscope according to claim 5, **characterised in that** the means for combining and splitting the beam paths comprise a pleochroitic, in particular a dichroitic, beam splitter (16).

7. Laryngoscope according to any one of the preceding claims,
**characterised in that** the OCT device further comprises an OCT module (30) which is attachable to the laryngoscope and has an OCT scanner and telescope optics.

8. Laryngoscope according to any one of the preceding claims,
**characterised by** means (14a-d) for influencing the depth of focus range of the observation device, in particular for matching the depth of focus range to the operating range of the OCT device.

9. Laryngoscope according to claim 8, **characterised in that** the means for influencing the depth of focus range can be deactivated.

10. Laryngoscope according to any one of claims 8 to 9, **characterised in that** the means for influencing the depth of focus range comprise an aperture in the imaging beam path and the size of said aperture is selected such that the depth of focus range of the observation device is substantially equal to the operating range of the OCT device, and in particular does not exceed the operating range of the OCT device.

11. Laryngoscope according to any one of claims 8 to 10, **characterised in that** the means for influencing the depth of focus range comprise means (31) for increasing the total magnification of the observation device, said means being selected such that the depth of focus range of the observation device is substantially equal to the operating range of the OCT device, and in particular does not exceed the operating range of the OCT device.

12. Laryngoscope according to claim 11, **characterised in that** means for increasing the total magnification are configured for continuous increase in total magnification.

13. Laryngoscope according to any one of the preceding claims,
**characterised by** means (31) for increasing the depth of focus range and/or the visual field of the observation device.

14. Laryngoscope according to claim 13, **characterised in that** the means for increasing the depth of focus range and/or the visual field of the observation device can be deactivated.

15. Laryngoscope according to any one of the preceding claims,
**characterised by** means for generating a visible pilot beam directed onto the area being examined by the OCT device.

16. Laryngoscope according to any one of the preceding claims,
**characterised in that** the second optical opening (31) cooperates with an image acquisition device (30), in particular a CCD camera.

17. Laryngoscope according to claim 16, **characterised by** an image reproduction device for displaying the image recorded by the image acquisition device.

18. Laryngoscope according to any one of the preceding claims,
**characterised in that** the second optical opening (31) is configured as an eyepiece to enable the examination area to be viewed directly by the eye of an examiner.

19. Laryngoscope according to any one of the preceding claims,
**characterised in that** the illumination beam path (13) can be connected to a stroboscope to illuminate the area being examined.

## Revendications

1. Laryngoscope comprenant
- un segment conducteur de lumière (11) pour introduction dans la cavité buccale d'un patient et
- un dispositif d'observation (14a-d, 31, 30) pour établir un diagnostic avec la lumière visible, comprenant
-- un parcours optique d'éclairage (13) pour éclairer une zone à examiner (2a, b) avec la lumière d'observation visible,
-- un parcours optique de représentation (13) pour conduire la lumière d'observation réfléchie de la zone à examiner,
-- une première ouverture optique (14a) pour l'entrée de la lumière d'observation réfléchie de la zone à examiner dans le laryngoscope, et
-- une deuxième ouverture optique (31) pour conduire la lumière d'observation réfléchie à un sujet réalisant l'examen,
- un dispositif OCT (14a-d, 16) pour établir un diagnostic par tomographie à cohérence optique, comprenant
-- un parcours optique d'éclairage à cohérence (13) pour éclairer une zone à examiner avec le rayonnement cohérent et
-- un parcours optique de représentation à cohérence (13) pour conduite le rayonnement cohérent réfléchi de la zone à analyser à un module OCT (20) pour générer un cliché planigraphique des tissus de la zone à examiner,
**caractérisé en ce que** l'intervalle de travail du dispositif OCT correspond à l'intervalle de travail du dispositif d'observation et en particulier, correspond à la distance entre la première ouverture optique du laryngoscope introduit dans la cavité buccale d'un patient et correspond aux cordes vocales du patient et la profondeur de champ du dispositif d'observation correspond essentiellement à la zone de travail du dispositif OCT.

2. Laryngoscope selon la revendication 1,
**caractérisé en ce que** le parcours optique d'éclairage (13) et le parcours optique de représentation (13) évoluent au moins dans la zone du segment (11) du laryngoscope introduit dans la cavité buccale de façon coaxiale.

3. Laryngoscope selon la revendication 1 ou 2,
**caractérisé en ce que** le parcours optique d'éclairage à cohérence (13) et le parcours optique de représentation à cohérence (13) évoluent au moins dans la zone du segment (11) du laryngoscope introduit dans la cavité buccale de façon coaxiale.

4. Laryngoscope selon l'une des revendications précédentes,
**caractérisé en ce qu'**au moins un parcours optique (13) du dispositif d'observation et au moins un parcours optique (13) du dispositif OCT évoluent au moins dans la zone du segment (11) du laryngoscope introduit dans la cavité buccale de façon coaxiale.

5. Laryngoscope selon l'une des revendications précédentes,
**caractérisé par** des moyens (16) de regroupement et de séparation d'au moins un parcours optique (13) du dispositif d'observation et au moins d'un parcours optique (13) du dispositif OCT.

6. Laryngoscope selon la revendication 5,
**caractérisé en ce que** les moyens de regroupement et de séparation des parcours optiques comprennent un séparateur de faisceau (16) pléochroïque, en particulier, dichroïque.

7. Laryngoscope selon l'une des revendications précédentes,
**caractérisé en ce que** le dispositif OCT comprend en outre un module OCT (30) pouvant être fixé au laryngoscope, associé à un scanner OCT et à une optique télescopique.

8. Laryngoscope selon l'une des revendications précédentes,
**caractérisé par** des moyens (14a-d) permettant d'influer sur la zone de profondeur de champ du dispositif d'observation, en particulier pour ajuster la profondeur de champ à la zone de travail du dispositif OCT.

9. Laryngoscope selon la revendication 8,
**caractérisé en ce que** l'action des moyens permettant d'influer sur la zone de profondeur de champ peut être neutralisée.

10. Laryngoscope selon l'une des revendications 8 à 9,
**caractérisé en ce que** les moyens permettant d'influer sur la zone de profondeur de champ comprennent une ouverture dans le parcours optique de représentation, dont la dimension est choisie de telle sorte que la zone de profondeur de champ du dispositif d'observation corresponde essentiellement à la zone de travail du dispositif OCT, en particulier, de sorte qu'elle ne soit pas plus grande que la zone de travail du dispositif OCT.

11. Laryngoscope selon l'une des revendications 8 à 10,
**caractérisé en ce que** les moyens permettant d'influer sur la zone de profondeur de champ comprennent des moyens (31) permettant d'augmenter l'agrandissement global du dispositif d'observation qui sont choisis de telle sorte que la zone de profondeur de champ du dispositif d'observation corresponde essentiellement à la zone de travail du dispositif OCT, en particulier, de sorte qu'elle ne soit pas plus grande que la zone de travail du dispositif OCT.

12. Laryngoscope selon la revendication 11,
**caractérisé en ce que** les moyens permettant d'augmenter l'agrandissement global sont conçus pour une augmentation continue de l'agrandissement global.

13. Laryngoscope selon l'une des revendications précédentes,
**caractérisé par** des moyens (31) d'agrandissement de la zone de profondeur de champ et/ou du champ visuel du dispositif d'observation.

14. Laryngoscope selon la revendication 13,
**caractérisé en ce que** l'action des moyens d'agrandissement de la zone de profondeur de champ et/ou du champ visuel du dispositif d'observation peut être neutralisée.

15. Laryngoscope selon l'une des revendications précédentes,
**caractérisé par** des moyens de génération d'un rayon pilote visible qui est dirigé sur la zone d'examen du dispositif OCT.

16. Laryngoscope selon l'une des revendications précédentes,
**caractérisé en ce que** la deuxième ouverture optique (31) coopère avec un dispositif de saisie d'image (30), en particulier, une caméra à dispositif de transfert de charge (CCD).

17. Laryngoscope selon la revendication 16,
**caractérisé par** un appareil de restitution d'image pour représenter l'image enregistrée avec l'appareil de saisie d'image.

18. Laryngoscope selon l'une des revendications précédentes,
**caractérisé en ce que** la deuxième ouverture optique (31) est conçue comme un oculaire pour pouvoir observer la zone d'examen directement à l'oeil nu d'un sujet réalisant l'examen.

19. Laryngoscope selon l'une des revendications précédentes,
**caractérisé en ce que** le parcours optique d'éclairage (13) peut être couplé à un stroboscope pour éclairer la zone d'examen.
